# FASCICULE DE BREVET EUROPEEN

(11) **EP 1 363 641 B1**
(45) Date de publication et mention de la délivrance du brevet: **26.10.2005**
(21) Numéro de dépôt: 02708426.8
(22) Date de dépôt: 27.02.2002
(51) Int. Cl.: A61K 31/57, A61K 9/16, A61P 15/12

(54) **PROGESTATIF CO-MICRONISE AVEC UN TENSIOACTIF, COMPOSITION PHARMACEUTIQUE LE COMPRENANT, LEURS PROCEDES DE FABRICATION ET LEURS UTILISATIONS**
MIT EINEM TENSID CO-MIKRONISIERTES PROGESTIN, PHARMAZEUTISCHE ZUSAMMENSETZUNG DIESES ENTHALTEND, VERFAHREN ZU DEREN HERSTELLUNG SOWIE VERWENDUNG
PROGESTIN CO-MICRONIZED WITH A SURFACTANT, PHARMACEUTICAL COMPOSITION COMPRISING THE SAME, METHODS FOR MAKING SAME AND USES THEREOF

(30) Priorité: 01.03.2001 FR 0102814
(43) Date de publication de la demande: 26.11.2003
(73) Titulaire: LABORATOIRES BESINS INTERNATIONAL, 75003 Paris (FR)
(72) Inventeur: BESSE, Jérôme, F-33480 Listrac Medoc (FR)
(74) Mandataire: Nargolwalla, Cyra
(86) Numéro de dépôt international: PCT/FR2002/000714
(87) Numéro de publication internationale: WO 2002/069978

(56) Documents cités:
- WO-A-00/28970
- US-A- 4 895 726
- US-A- 6 063 404

## Description

La présente invention a pour objet, en tant que produit nouveau, un progestatif co-micronisé avec un tensioactif, une composition pharmaceutique le contenant, des procédés pour leur préparation ainsi que leurs utilisations.

Dans le contexte de la présente invention, on entend par « progestatif » tout stéroïde doué d'affinités pour les récepteurs à la progestérone et capable de reproduire de façon plus ou moins complète les effets biologiques de la progestérone.

Un progestatif est un composé capable, par définition, de maintenir la gestation et de favoriser l'implantation de l'oeuf. Ce rôle biologique se traduit essentiellement par une modification de la muqueuse vaginale (desquamation), de l'endomètre (prolifération cellulaire, formation de la dentelle utérine) et de l'épithélium glandulaire endocervical (diminution de la production de glaire et épaississement de celle-ci).

La seule propriété que toutes les substances progestatives ont en commun est l'action endométriale.

L'effet sur la gestation est réel pour la progestérone et très inconstant avec les progestatifs de synthèse.

Les progestatifs comprennent la progestérone ainsi que les progestatifs de synthèse. Ces derniers peuvent se classer en trois groupes (classification non officielle) selon leurs activités biologiques (et leur structure, celle-ci déterminant celles-là) ; l'ordre de classement rend donc compte de leur éloignement structural par rapport à la progestérone physiologique.

Le premier groupe comprend les molécules proches de la progestérone ou les progestatifs de synthèse 1 (PS1) (pregnanes), par exemple, l'isomère de progestérone (rétroprogestérone), le Medrogestérone, les dérivés de la norprogestérone (démégestone ou promégestone). Ces molécules ont une activité extra-gestative périphérique pratiquement semblable à la progestérone et n'ont pas d'effet androgénique.

Le second groupe comprend les dérivés de la 17α-hydroxy-progestérone ou progestatifs de synthèse 2 (PS2) (pregnanes), par exemple l'acétate de cyprotérone, l'acétate de médroxyprogestérone. Ces molécules ont une activité gestative périphérique plus puissante et plus intense que celle de la progestérone et ont en plus un effet androgénique.

Le troisième groupe comprend les norstéroides ou progestatifs de synthèse 3 (PS3), (estranes ou nor-androstanes). Ce sont des dérivés de la 19-nortestostérone, par exemple la noréthindrone. Ces molécules ont une activité gestative périphérique particulièrement puissante (il s'agit du groupe de progestatifs de synthèse qui a l'action endométriale la plus prononcée)et ont également un effet androgénique. De ces nor-androstanes ou estranes dérivent les molécules de type gonane présentant un groupe méthyle en C18 et un groupe éthyle en C13. On peut citer comme exemples le norgestimate (levonorgestrel), le désogestrel (3-céto désogestrel) et le gestodène. Ces modifications chimiques augmentent le pouvoir endrométrial et diminuent l'activité androgénique intrinsèque de la molécule.

La progestérone est une hormone qui est synthétisée, chez la femme, essentiellement par l'ovaire lors de la phase post-ovulaire ou lutéale (corps jaune) et, à un moindre degré, par les glandes surrénales et le placenta au cours de la deuxième partie de la grossesse. Une synthèse non endocrine de la progestérone, notamment au niveau des neurones, est également possible.

L'insuffisance de la sécrétion de la progestérone chez une femme a pour conséquence une perte de ses effets biologiques : effet progestatif, effet anti-androgène (action sur la peau) et anti-oestrogène (avec pour conséquence une hyperoestrogénie : bouffées de chaleur, difficultés psychogènes de type anxieux ou dépressif, prise de poids,...). Cette insuffisance en progestérone peut conduire à des gênes fonctionnelles et des manifestations cliniques diverses, en particulier:
- syndromes prémenstruels,
- irrégularités menstruelles par disovulation ou anovulation,
- mastopathies bénignes,
- périménopause et ménopause.

Pour le soulagement des symptômes ménopausiques, l'emploi de l'hormonothérapie substitutive est bien établi à ce jour. Etant donné qu'il est démontré que les progestatifs préviennent le développement de l'hyperplasie et du cancer de l'endomètre, une thérapie séquentielle ou combinée d'oestrogènes et de progestatifs est conseillée chez les femmes ménopausées non hystérectomisées. Parmi les progestatifs adaptés à l'hormonothérapie substitutive, la progestérone micronisée est de préférence utilisée du fait de son absence d'effet androgène et de son innocuité métabolique.

Mais l'administration par voie orale de la progestérone souffre d'un grave handicap en raison de sa métabolisation importante par le foie.

Or, le mode d'administration par voie orale présente des avantages déterminants par rapport à d'autres méthodes d'administration. En effet, elle est d'une part plus pratique que l'administration par voie vaginale, et d'autre part, elle permet une prise indépendante qui est impossible lorsqu'il s'agit de l'administration par voie parentérale.

Les LABORATOIRES BESINS-ISCOVESCO ont déjà proposé une solution à ce problème de dégradation de la progestérone, dans la demande de brevet FR 76 36007. En effet, ils ont développé une formulation de capsules molles contenant de la progestérone micronisée en suspension huileuse, ce qui permettait une amélioration de la biodisponibilité de la progestérone.

Le procédé de préparation de telles capsules se révèle cependant d'une mise en oeuvre complexe et coûteuse, nécessitant en plus un savoir faire considérable. On a donc essayé de développer des formulations alternatives efficaces, mais également économiquement viables, comme des comprimés à base de progestérone (voir les demandes de brevet FR 97 16168 et FR 98 02830).

Cependant, il existe toujours un besoin de trouver des formulations pharmaceutiques contenant de la progestérone ou un autre progestatif et ayant une biodisponibilité améliorée.

De façon générale, la biodisponibilité d'un principe actif peut être améliorée par des moyens chimiques : administration de pro-drugs, complexation, association avec des lipides ou des phospholipides, le plus souvent en présence d'un tensioactif ; ou par des moyens physiques comme la micronisation.

La micronisation est une technique bien connue, qui peut être réalisée soit dans des broyeurs à marteaux ou à boulets ou dans des micronisateurs à jets gazeux. Comme cela a été rappelé précédemment, la technique de micronisation a déjà été utilisée (voir brevet BESINS FR 76 36007) pour mettre au point une composition à base de progestérone biodisponible et administrable par voie orale.

La demande de brevet FR 2 757 397 rappelle également ce fait et indique ensuite, en page 2, que la co-micronisation du fénofibrate en présence de laurylsulfate de sodium a été décrite dans le brevet EP 330 532. Mais cependant, cette demande de brevet souligne en même temps le fait qu'il n'est pas automatique et inéluctable que la biodisponibilité d'un principe actif soit systématiquement améliorée par une co-micronisation en présence d'un tensioactif. C'est ainsi que les inventeurs de cette demande de brevet rappellent que M. OTSUDA et al. (JPS 84, 1995, p. 1434-37) ont étudié la micronisation de la phénitoïne en présence d'un agent tensioactif et qu'ils ont montré que la solubilité de la phénitoïne n'est pas améliorée dans le cas de la co-micronisation avec un tensioactif tel que le laurylsulfate de sodium ou un sucrose-ester d'acide stéarique, tandis qu'elle est multipliée par 30 par rapport au mélange des poudres dans le cas d'un cobroyage avec le désoxycholate de sodium.

Les mêmes auteurs ont souligné par ailleurs - ce qui est tout à fait pertinent - que même si la micronisation ou le broyage d'une substance en présence d'un tensioactif ou d'un sucre peut augmenter sa solubilité, ces paramètres ne sont pas toujours suffisants. Ils ont ainsi donné pour exemple le fait que la biodisponibilité de la progestérone micronisée n'est pas satisfaisante et qu'elle doit être améliorée, par exemple par mélange de la progestérone micronisée avec de la cire de carnauba, technique décrite dans la demande de brevet WO 89/02742.

Il est donc parfaitement établi que les propriétés d'une substance traitée par micronisation ou broyage, en particulier sa solubilité et sa biodisponibilité, ne sont pas prévisibles, des résultats contradictoires pouvant être obtenus, et qu'une même formulation galénique peut procurer de bons résultats avec une substance et aboutir à un résultat contraire avec une autre substance.

Or, il est du mérite de la Société Demanderesse d'avoir réussi à améliorer non seulement la solubilité mais également la biodisponibilité des progestatifs, dont tout particulièrement la progestérone, en procédant à leur co-micronisation en présence d'un tensioactif, ce qui n'était ni enseigné ni même suggéré par les documents de l'art antérieur.

L'invention a donc pour objet, en tant que produit nouveau, un progestatif co-micronisé avec un tensioactif, ainsi qu'une composition pharmaceutique contenant ledit progestatif co-micronisé. L'invention a également pour objets le procédé de fabrication du progestatif co-micronisé, le procédé de fabrication de la composition pharmaceutique comprenant le progestatif co-micronisé, ainsi que l'utilisation dudit progestatif pour la préparation d'un médicament destiné à prévenir ou soigner les troubles ou maladies occasionnés par une insuffisance de progestatifs.

On entend, au sens de la présente invention, par le terme de tensioactif, tout produit présentant à la fois une partie lipophile et une partie hydrophile. Ces produits sont généralement classés comme ioniques ou non ioniques. On préfère utiliser, conformément à la présente invention, un tensioactif ionique. Plus préférentiellement encore, on utilise le laurylsulfate de sodium.

La co-micronisation de la progestérone ou d'un autre progestatif avec un tensioactif, en particulier le laurylsulfate de sodium, permet une amélioration de la solubilité du principe actif, permettant l'obtention de profils de dissolution in vitro rapides. La co-micronisation permet également d'améliorer la mouillabilité du progestatif lors de son administration per os, facilitant ainsi son absorption in vivo.

Selon un mode de réalisation avantageux de l'invention, le progestatif co-micronisé est la progestérone et le tensioactif est le laurylsulfate de sodium.

La teneur en tensioactif est au moins égale à la concentration micellaire critique de l'association progestatif / tensioactif.

Le progestatif co-micronisé conforme à l'invention présente une teneur en progestatif comprise entre 80,0 % et 99,9 %, de préférence entre 90,0 % et 99,5 %, et plus préférentiellement encore entre 95,0 % et 99,0 % et une teneur en tensioactif comprise entre.0,1 % et 20,0 %, de préférence entre 0,5 % et 10,0 % et plus préférentiellement encore entre 1,0 % et 5,0 %, les pourcentages étant exprimés en poids de matière sèche.

Dans le cas où le progestatif est la progestérone, le rapport tensioactif / progestérone est compris entre 1/200 et 1/20, de préférence entre 1/150 et 1/40.

L'invention a également pour objet une composition pharmaceutique comprenant le progestatif co-micronisé avec un tensioactif tel que décrit ci-dessus.

Selon un mode de réalisation avantageux, la composition pharmaceutique conforme à l'invention présente une teneur en tensioactif comprise entre 0,001 % et 5 %, de préférence entre 0,002% et 3%, et plus préférentiellement encore entre 0,005% et 2% par rapport à la matière sèche totale.

Dans le cas où le progestatif co-micronisé est la progestérone, la teneur en tensioactif est alors comprise entre 0,1% et 5%, de préférence entre 0,2% et 3% , plus préférentiellement encore entre 0,2% et 2% en poids par rapport à la matière sèche totale de la composition pharmaceutique.

La composition pharmaceutique selon la présente invention peut se présenter sous la forme par exemple d'une capsule molle, d'une gélule, d'un comprimé, d'un lyophilisat, d'une poudre ou d'une granule, y compris des microgranules. De préférence, la composition pharmaceutique selon l'invention est un comprimé.

Lorsqu'il s'agit d'un comprimé, la composition pharmaceutique selon l'invention contient des excipients permettant notamment de faciliter la compression afin d'obtenir un comprimé possédant de bonnes caractéristiques de dureté, de désagrégation et de dissolution.

Comme excipients susceptibles d'être utilisés dans le comprimé selon l'invention, on peut citer les agents diluants, les agents désintégrants, les agents lubrifiants, les agents liants et les colorants conventionnellement utilisés dans cette application.

Comme exemples d'agents diluants on peut citer les sucres, les amidons, les polyols et les celluloses et dérivés. De préférence, le comprimé selon l'invention contient du lactose et/ou du mannitol en tant qu'agents diluants.

Comme exemples d'agents désintégrants on peut citer les carboxyméthylcelluloses, l'acide alginique ainsi que son sel de sodium, et les amidons modifiés. De préférence, le comprimé selon l'invention contient de la carboxyméthylcellulose sodique réticulée (aussi appelée croscarmellose sodique).

L'agent lubrifiant préféré utilisé dans le cadre de la présente invention est le stéarate de magnésium.

Parmi les agents liants préférés dans le cadre de la fabrication du comprimé selon la présente invention, on peut citer les polyvinylpyrrolidones. De préférence, le comprimé selon l'invention contient la polyvidone K30.

Le comprimé selon l'invention peut également contenir un colorant, tel que par exemple le colorant jaune orangé S ou le Pigment Blend PB 23028.

La composition pharmaceutique selon l'invention peut également contenir un oestrogène ou un de ces dérivés.

En effet, les oestrogènes, particulièrement le 17-β-estradiol, sont prescrits afin de réduire les conséquences néfastes liées à leur disparition au cours de la ménopause, telles que l'ostéoporose, les bouffées de chaleur ou les accidents cardio-vasculaires. L'administration des oestrogènes seuls à long terme présente des risques majeurs compte tenu notamment du rôle carcinogène éventuel de cette hormone. Il est habituel d'y associer un traitement avec un progestatif afin d'éviter les risques d'hyperplasie de l'endomètre.

L'oestrogène pouvant être inclus dans la composition pharmaceutique selon la présente invention peut être sélectionné parmi les oestrogènes actifs per os c'est à dire les oestrogènes naturels (17-β-estradiol, estrone), ou synthétiques (17α-éthinyl-estradiol ou valérate d'estradiol). De préférence on utilise le 17-β estradiol.

La teneur en oestrogène de la composition pharmaceutique selon l'invention est comprise entre 0,05% et 5%, de préférence entre 0,1% et 3%.

L'invention a également pour objet un procédé de co-micronisation d'un progestatif avec un tensioactif. Ce co-micronisat peut être fabriqué à partir d'un mélange solide/solide (comme dans le cas du mélange progestérone/laurylsulfate de sodium) à l'aide d'un broyeur à jets d'air, tel que celui commercialisé sous la marque ALPINE.

Dans le cas d'un co-micronisat solide/liquide (exemple : progestérone/TWEEN 80), l'opération de co-micronisation peut être réalisée avec un broyeur colloïdal ou encore un broyeur à boulets.

Le procédé de préparation du progestatif co-micronisé avec un tensioactif est donc caractérisé par le fait que l'on prépare un mélange d'un progestatif avec un tensioactif, que l'on effectue ensuite soit une étape de broyage avec un broyeur à jets d'air, dans le cas d'une co-micronisation solide/solide, soit une étape de broyage avec un broyeur colloïdal ou un broyeur à boulets, dans le cas d'une co-micronisation solide/liquide.

Dans le cas où la composition pharmaceutique selon l'invention se trouve sous forme d'un comprimé, le procédé de préparation d'un comprimé à base de progestatif comporte, de façon préférentielle, les étapes suivantes :
- préparation d' un premier mélange de progestatif et de tensioactif,
- micronisation de ce premier mélange afin d'obtenir un progestatif co-micronisé avec le tensioactif
- préparation d'une solution de mouillage
- mouillage du progestatif co-micronisé avec le tensioactif par la solution de mouillage,
- granulation du mélange obtenu afin d'obtenir des granules,
- séchage des granules puis calibrage,
- ajout aux granules calibrés des agents désintégrants, diluants, colorants, lubrifiants,
- compression du mélange ainsi obtenu afin d'obtenir des comprimés.

L'invention porte également sur l'utilisation d'un progestatif co-micronisé avec un tensioactif tel que défini ci-dessus, pour la préparation d'un médicament destiné au traitement d'une condition physiologique liée à l'insuffisance de la sécrétion de la progestérone, telle que la ménopause.

L'invention sera mieux comprise à l'aide des exemples non-limitatifs décrits ci-dessous.

### EXEMPLE 1 : PREPARATION DE PROGESTERONE CO-MICRONISEE AVEC LE LAURYLSULFATE DE SODIUM

1) On mélange, pendant 5 minutes, dans un mélangeur de type LÖDIGE, 97g de progestérone et 3g de laurylsulfate de sodium.
2) On introduit le mélange obtenu à l'étape (1) dans un broyeur à jet d'air Alpine 200 AS préalablement réglé sur les paramètres suivants :
   - Injection : 5,5 B
   - Couronne : 3,0 B
   - Vitesse : 35 kg/h
3) Ce mélange est broyé afin d'obtenir un co-micronisat de progestérone/laurylsulfate de sodium.

### EXEMPLE 2 : COMPRIMES A BASE DE PROGESTERONE CO-MICRONISEE AVEC UN TENSOACTIF

Les formulations de comprimés de progestérone selon l'invention, contenant 100 et 200 mg de progestérone, sont données dans le Tableau I ci-dessous.

**TABLEAU I**

| NOM DU COMPOSANT | FONCTION | QUANTITE UNITAIRE mg/comprimé | | TAILLE DU LOT (kg) |
|---|---|---|---|---|
| Progestérone* micronisée | Principe actif (progestatif) | 200 | 100 | 55,000 |
| (Laurylsulfate de sodium (SLS))* | (Tensioactif) | 6,18 | 3,09 | |
| Povidone K30 Solution à 35% (m/m) | Liant | 9,60 | 4,80 | 2,561 |
| Mannitol | Diluant | 29,84 | 14,92 | 7,981 |
| Carboxyméthyl cellulose sodique réticulée | Désintégrant | 13,00 | 6,50 | 3,468 |
| Stéarate de magnésium | Lubrifiant | 1,30 | 0,65 | 0,347 |

| | | | | |
|---|---|---|---|---|
| * Progestérone/SLS sont co-micronisés suivant l'exemple 1. | | | | |

Pour préparer les comprimés en question, il a été procédé comme suit :
Etape 1 : PREPARATION DE LA SOLUTION DE MOUILLAGE
On introduit 4,756 kg d'eau purifiée dans un récipient de capacité adaptée. On introduit ensuite 2,561 kg de Povidone K30 dans l'eau purifiée, progressivement, et sous agitation dans un agitateur de type défloculeuse (RAYNERI) à une vitesse d'agitation d'environ 1800 tr/minute et pendant 30 minutes jusqu'à solubilisation complète de la Povidone K30.
Etape 2 : GRANULATION
On introduit dans la cuve d'un mélangeur du type LÖDIGE, 55,000 kg de Progestérone co-micronisée.
On mouille le mélange précédent avec les solutions de l'étape 2, et puis on effectue une granulation jusqu'à l'obtention d'un aspect visuel satisfaisant.
Les conditions de la granulation sont les suivantes :

| | |
|---|---|
| Quantité d'eau purifiée ajoutée en QS Aspect final du grain satisfaisant % d'humidité résiduelle | 6,8 |
| Durée de mouillage (environ 1 min) | 30 sec |
| Durée de granulation (Soc + couteaux) | 5 min 30 sec |
| Puissance absorbée (fin granulats) | (environ 38 %) |

Un démottage humide du grain en sortie du granulateur peut être réalisé si nécessaire avant séchage sur un calibreur rotatif ALEXANDERWERK ou équivalent équipé d'une grille en acier inoxydable de diamètre supérieur ou égal à 3 mm.
Etape 3 : SECHAGE
On sèche le grain de l'étape 2 jusqu'à l'obtention d'une humidité résiduelle satisfaisante.
Les conditions de séchage sont les suivantes :
- Température air entrée (environ 55°C)
- Température air sortie : 39°C
   Fin à 42°C (en fin de séchage)
- Durée : 10 min

Etape 4 : CALIBRAGE
On calibre le grain de l'étape 3 sur BOHLE équipé d'une grille d'ouverture de maille 1,0 mm ou équivalent.
Les conditions de calibrage sont les suivantes :
- Durée : 12 min
- Quantité obtenue : 55,040 kg
- % humidité résiduelle du grain calibré.

On homogénéise pendant 5 min à 5 tr/min en BOHLE ou équivalent.
Etape 5 : MELANGE
On introduit dans la cuve MCG 600 du mélangeur BOHLE PM 100 de capacité adaptée ou équivalent :
- le grain calibré de l'étape 4 : 54,740 kg
- la croscarmellose : 3,298 kg
- le mannitol : 7,590 kg

On mélange à une vitesse de 5 tr/min pendant environ 30 min.
Etape 6 : LUBRIFICATION
On introduit dans le mélange de l'étape 5 0,330kg de stéarate de magnésium :
On mélange à une vitesse de 5 tr/min pendant environ 10 min.
Le poids net est de 64,490 kg.
Le mélange est stocké dans un container étanche.

Etape 7 : COMPRESSION
On équipe la machine à comprimer de type KILIAN RTS 21 ou équivalent avec les poinçons de format type 7R7,5 pour Progestérone 100 mg et 9R10 pour Progestérone 200 mg.
On alimente la trémie de la machine à comprimer avec le mélange de l'étape 6.
On procède aux réglages de compression de manière à obtenir des comprimés de masse 130 mg de dureté et épaisseur satisfaisantes permettant de garantir un temps de délitement inférieur à 5 min.

### EXEMPLE 3: DISSOLUTION IN VITRO DES COMPRIMES DE PROGESTERONE PREPARES SELON L'INVENTION

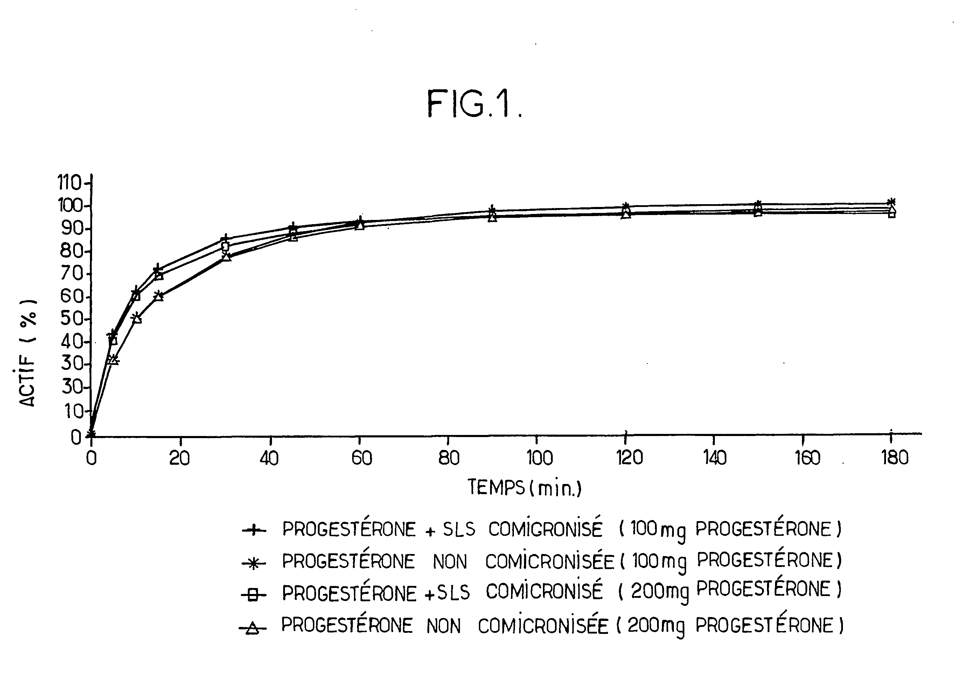

On prépare des comprimés de progestérone de 100 et de 200 mg selon la méthode donnée à l'exemple 2, et on effectue les opérations suivantes afin de déterminer les courbes de dissolution in vitro (voir Figure 1).

On utilise le matériel suivant :
- Appareil de dissolution à pales tournantes 7 postes SOTAX AT7
- Spectrophotomètre PERKIN ELMER Lambda 20
- Pompe à cassettes ISMATEC IPC 12
- Logiciel d'acquisition de données WINSOTAX
Les conditions de dissolution sont les suivantes :
- Milieu de dissolution : 1000 ml de solution aqueuse de β-hydroxypropylcyclodextrine de marque KLEPTOSE® à 1 % par cuve
   - Vitesse de rotation : 150 rpm
   - Température : 37° C ± 0,5° C
   - Nombre de cuves : 7
- Cuve de circulation en quartz de trajet optique : 0,1 cm

On prépare un témoin constitué par un comprimé de progestérone micronisée:

| | |
|---|---|
| Progestérone | 20 mg |
| Ethanol CLHP | 2 ml |
| KLEPTOSE® solution à 1 % qsp | 200 ml |

L'agitation est conduite à l'aide de paniers tournants.

### Mode opératoire

1 - On place les cuves dans le bain-marie à température ambiante.
2 - On transfère 1000 ml de solution de KLEPTOSE® à 1 % dans chacune des 7 cuves.
3 - On place 1 comprimé dans 6 des 7 paniers tournants. La cuve 7 est utilisée comme référence en cours d'essai.
4 - On immerge les paniers tournants dans le milieu de dissolution à une distance de 25 mm ± 2 mm entre le panier tournant et le fond de la cuve.
5 - On met les paniers sous une agitation de 150 rpm.
6 - A chaque intervalle de temps prévu, on prélève automatiquement dans chacune des 7 cuves la quantité de milieu nécessaire et suffisante pour la détermination de la concentration en principe actif dissous. Cette mesure est réalisée avec l'appareil décrit ci-dessus.
7 - Chaque échantillon collecté est dosé par spectrophotométrie (λ = 248 nm).
8 - On détermine le pourcentage de progestérone libérée.

Les résultats sont donnés dans le tableau II ci-dessous ainsi que dans la Figure 1.

### Légende :

GAL 207.07 : Comprimé Progestérone 100 mg lot 102 (co-micronisation Progestérone/laurylsulfate de sodium)

GAL 208.03 : Comprimé Progestérone 200 mg lot 101 (co-micronisation Progestérone/laurylsulfate de sodium)

GAL 207.08 : Comprimé Progestérone 100 mg principe actif non co-micronisé lot 00VR1108.01

GAL 208.04 : Comprimé Progestérone 200 mg principe actif non co-micronisé lot 00VR1110.01

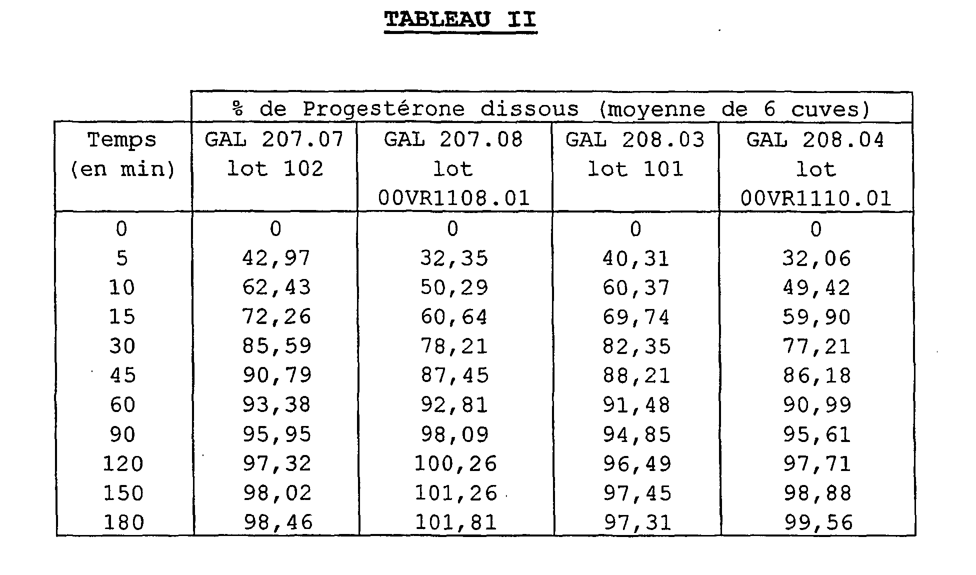

### EXEMPLE 4: COMPRIMES SELON L'INVENTION CONTENANT UN PROGESTATIF COMICRONISE AVEC UN TENSIOACTIF

Les formulations de comprimés selon l'invention contenant soit de la progestérone, soit du lévonogestrel, sont données dans les tableaux III à V ci-dessous.

**TABLEAU III**

| **NOM DU COMPOSANT** | **FONCTION** | **QUANTITE UNITAIRE mg/comprimé** | **% COMPRIME** |
|---|---|---|---|
| Progestérone* micronisée | Principe actif progestatif | 100 | 77,00 |
| LUTROL® F127* (Poloxamère 407) | Tensioactif (poloxamer) | 3,09 | 2,30 |
| Kollidonr® 30 (Povidone K30) | Liant | 8,31 | 6,39 |
| Pearlitol® 500DC (Mannitol) | Diluant | 11,45 | 8,81 |
| AcDiSol® (Crosscarmellose) | Désintégrant | 6,50 | 5,00 |
| Stéarate de magnésium | Lubrifiant | 0,65 | 0,50 |

| | | | |
|---|---|---|---|
| * Progestérone/Lutrol sont co-micronisés suivant le protocole cité dans l'exemple 1. | | | |

**TABLEAU IV**

| **NOM DU COMPOSANT** | **FONCTION** | **QUANTITE UNITAIRE mg/comprimé** | **% COMPRIME** |
|---|---|---|---|
| Lévonorgestrel* | Principe actif progestatif | 0,75 | 0,58 |
| Laurylsulfate de sodium (SLS) | Tensioactif | 0,023 | 0,02 |
| Mannitol 60 | Diluant | 102,303 | 78,69 |
| Kollidon® 30 (Povidone K30) | Liant | 4,194 | 3,23 |
| Pearlitol® 500DC (Mannitol) | Diluant | 15,58 | 11,98 |
| AcDiSol® (Crosscarmellose) | Désintégrant | 6,50 | 5,00 |
| Stéarate de magnésium | Lubrifiant | 0,65 | 0,50 |

| | | | |
|---|---|---|---|
| * Lévonorgestrel/SLS sont co-micronisés suivant le protocole cité dans l'exemple 1. | | | |

**TABLEAU V**

| **NOM DU COMPOSANT** | **FONCTION** | **QUANTITE UNITAIRE mg/comprimé** | **% COMPRIME** |
|---|---|---|---|
| Lévonorgestrel* | Principe actif Progestatif | 0,75 | 0,58 |
| Lutrol® F127 (Poloxamère 407) | Tensioactif | 0,023 | 0,02 |
| Mannitol 60 | Diluant | 102,3 | 78,69 |
| Kollidon® 30 (Povidone K30) | Liant | 4,194 | 3,23 |
| Pearlitol® 500DC (Mannitol) | Diluant | 15,58 | 11,98 |
| AcDiSol® (Crosscarmellose) | Désintégrant | 6,50 | 5,00 |
| Stéarate de Magnésium | Lubrifiant | 0,65 | 0,50 |

| | | | |
|---|---|---|---|
| * Lévonorgestrel/Lutrol sont co-micronisés suivant le protocole cité dans l'exemple 1. | | | |

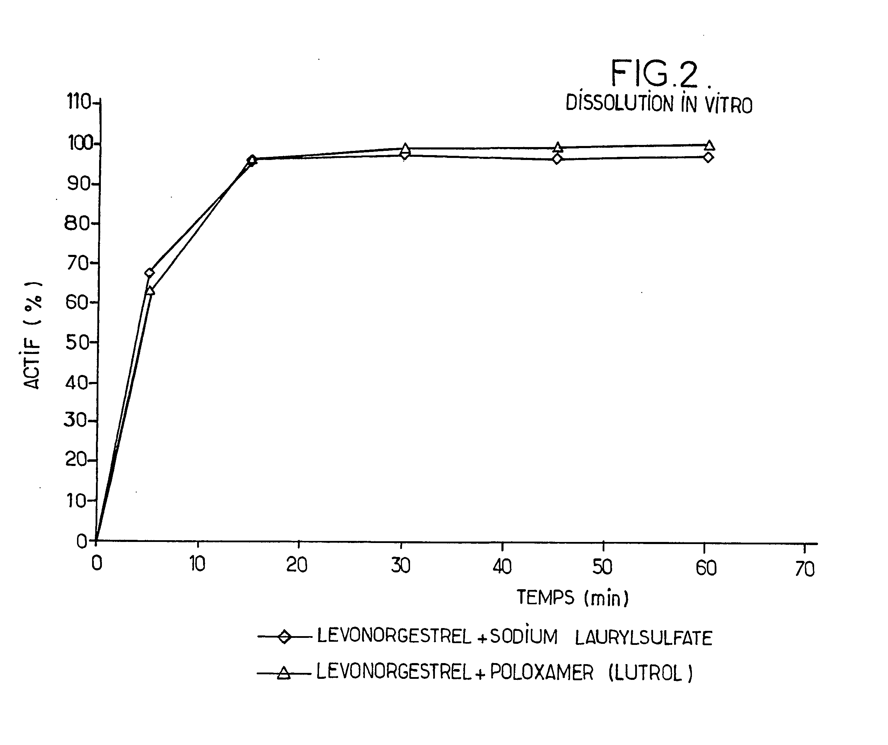

La figure 2 montre des essais de dissolution in vitro des formulations données dans les Tableaux IV et V.

Ces courbes démontrent que le principe actif co-micronisé est libéré de façon très satisfaisante.

## Revendications

1. Progestatif co-micronisé avec un tensioactif.

2. Progestatif co-micronisé selon la revendication 1, **caractérisé par le fait que** le progestatif est la progestérone.

3. Progestatif co-micronisé selon l'une ou l'autre des revendications 1 et 2, **caractérisé par le fait que** le tensioactif est le laurylsulfate de sodium.

4. Progestatif co-micronisé selon l'une quelconque des revendications 1 à 3, **caractérisé par le fait qu'**il présente une teneur en progestatif comprise entre 80,0 % et 99,9 %, de préférence entre 90,0 % et 99,5 %, et plus préférentiellement encore entre 95,0 % et 99,0 % et une teneur en tensioactif comprise entre.0,1 % et 20,0 %, de préférence entre 0,5 % et 10,0 % et plus préférentiellement encore entre 1,0 % et 5,0 %, les pourcentages étant exprimés en poids de matière sèche.

5. Progestérone co-micronisée avec un tensioactif, de préférence le laurylsulfate de sodium, **caractérisée par le fait que** le rapport tensioactif /progestérone est compris entre 1/200 et 1/20, de préférence entre 1/150 et 1/40.

6. Composition pharmaceutique comprenant le progestatif co-micronisé avec un tensioactif selon l'une quelconque des revendications 1 à 4.

7. Composition pharmaceutique comprenant la progestérone co-micronisée avec un tensioactif selon la revendication 5.

8. Composition pharmaceutique selon l'une ou l'autre des revendications 6 et 7, **caractérisée par le fait qu'**elle présente une teneur en tensioactif comprise entre 0,001 % et 5 %, de préférence entre 0,002 % et 3 %, et plus préférentiellement encore entre 0,005 % et 2% par rapport à la matière sèche totale de la composition.

9. Composition pharmaceutique selon la revendication 7, **caractérisée par le fait qu'**elle présente une teneur en tensioactif comprise entre 0,1% et 5%, de préférence entre 0,2% et 3% , plus préférentiellement encore entre 0,2% et 2% en poids par rapport à la matière sèche totale de la composition.

10. Composition pharmaceutique selon l'une quelconque des revendications 6 à 9, **caractérisée par le fait qu'**elle se trouve sous forme de capsule molle, de gélule, de comprimé, ou de lyophilisat, de poudre ou de granule, y compris microgranules.

11. Composition pharmaceutique selon l'une quelconque des revendications 6 à 10, **caractérisée par le fait qu'**elle contient, outre le progestatif co-micronisé, un oestrogène ou un de ces dérivés.

12. Composition pharmaceutique selon la revendication 11, **caractérisée par le fait que** l'oestrogène est sélectionné dans le groupe constitué par le 17-β estradiol, l'estrone, le 17α-éthinyl-estradiol et le valérate d'estradiol, de préférence le 17-β estradiol.

13. Composition pharmaceutique selon l'une ou l'autre des revendications 11 et 12, **caractérisée par le fait qu'**elle présente une teneur en oestrogène comprise entre 0,05% et 5%, de préférence entre 0,1% et 3%.

14. Procédé de co-micronisation d'un progestatif avec un tensioactif, **caractérisé par le fait que** l'on prépare un mélange d'un progestatif avec un tensioactif et que l'on effectue ensuite un broyage de ce mélange à l'aide d'un broyeur à jets d'air, d'un broyeur colloïdal ou d'un broyeur à boulets.

15. Procédé de préparation d'un comprimé à base d'un progestatif, de préférence la progestérone,
**caractérisé par le fait que** :
- on prépare un premier mélange de progestatif et de tensioactif,
- on micronise ce premier mélange afin d'obtenir un progestatif co-micronisé avec le tensioactif,
- on prépare une solution de mouillage,
- on procède au mouillage du progestatif co-micronisé avec le tensioactif par la solution de mouillage,
- on procède à une granulation du mélange obtenu afin d'obtenir des granules,
- on sèche les granules puis on les calibre,
- on ajoute aux granules séchés et calibrés des agents désintégrants, diluants, colorants, lubrifiants,
- on effectue une compression de ce mélange afin d'obtenir des comprimés.

16. Procédé de préparation selon la revendication 15, **caractérisé par le fait que** le tensioactif est le laurylsulfate de sodium.

17. Utilisation d'un progestatif co-micronisé avec un tensioactif selon l'une quelconque des revendications 1 à 4 ou de la progestérone co-micronisée avec un tensioactif selon la revendication 5, pour la préparation d'un médicament destiné au traitement d'une condition physiologique liée à l'insuffisance de la sécrétion de la progestérone, telle que la ménopause.

## Patentansprüche

1. Mit einem Tensid co-mikronisiertes Progestin.

2. Co-mikronisiertes Progestin nach Anspruch 1, **dadurch gekennzeichnet, dass** das Progestin Progesteron ist.

3. Co-mikronisiertes Progestin nach einem der Ansprüche 1 und 2, **dadurch gekennzeichnet, dass** das Tensid Natriumlaurylsulfat ist.

4. Co-mikronisiertes Progestin nach einem der Ansprüche 1 bis 3, **dadurch gekennzeichnet, dass** es einen Gehalt an Progestin von 80,0 % bis 99,9 %, vorzugsweise von 90,0 % bis 99,5 %, noch stärker bevorzugt von 95,0 % bis 99,0 % und einen Tensidgehalt von 0,1 % bis 20,0 %, vorzugsweise von 0,5 % bis 10,0 % und noch stärker bevorzugt von 1,0 % bis 5,0 % aufweist, wobei die Prozentsätze auf Trockenmaterialgewicht bezogen sind.

5. Mit einem Tensid, vorzugsweise mit Natriumlaurylsulfat co-mikronisiertes Progesteron, **dadurch gekennzeichnet, dass** das Verhältnis Tensid/Progesteron von 1/200 bis 1/20, vorzugsweise von 1/150 bis 1/40 beträgt.

6. Pharmazeutische Zusammensetzung, umfassend das mit einem Tensid co-mikronisierte Progestin nach einem der Ansprüche 1 bis 4.

7. Pharmazeutische Zusammensetzung, umfassend das mit einem Tensid co-mikronisierte Progesteron nach Anspruch 5.

8. Pharmazeutische Zusammensetzung nach einem der Ansprüche 6 und 7, **dadurch gekennzeichnet, dass** sie einen Tensidgehalt von 0,001 % bis 5 %, vorzugsweise von 0,002 bis 3 % und noch stärker bevorzugt von 0,005 % bis 2 % aufweist, bezogen auf den Gesamttrockenmaterialgehalt der Zusammensetzung.

9. Pharmazeutische Zusammensetzung nach Anspruch 7, **dadurch gekennzeichnet, dass** sie einen Tensidgehalt von 0,1 % bis 5 Gew.%, vorzugsweise von 0,2 % bis 3 Gew.%, noch stärker bevorzugt von 0,2 % bis 2 Gew.%, bezogen auf den Gesamttrockenmaterialgehalt der Zusammensetzung, aufweist.

10. Pharmazeutische Zusammensetzung nach einem der Ansprüche 6 bis 9, **dadurch gekennzeichnet, dass** sie in Form einer Weichkapsel, einer Kapsel, einer Tablette, eines Lyophilisats, eines Pulvers oder eines Granulats, einschließlich Mikrogranulate, vorliegt.

11. Pharmazeutische Zusammensetzung nach einem der Ansprüche 6 bis 10, **dadurch gekennzeichnet, dass** sie außer dem co-mikronisierten Progestin ein Estrogen oder ein Derivat davon enthält.

12. Pharmazeutische Zusammensetzung nach Anspruch 11, **dadurch gekennzeichnet, dass** das Estrogen aus der aus 17-β-Estradiol, Estron, 17α-Ethinylestradiol und Estradiolvalerat bestehenden Gruppe ausgewählt ist, vorzugsweise das 17-β-Estradiol.

13. Pharmazeutische Zusammensetzung nach einem der Ansprüche 11 und 12, **dadurch gekennzeichnet, dass** sie einen Estrogengehalt von 0,05 % bis 5 %, vorzugsweise von 0,1 % bis 3 % aufweist.

14. Verfahren zur Co-Mikronisierung eines Progestins mit einem Tensid, **dadurch gekennzeichnet, dass** man ein Gemisch eines Progestins mit einem Tensid bereitet und anschließend dieses Gemisch mit Hilfe einer Luftstrahlmühle, einer Kolloidmühle oder einer Kugelmühle mahlt.

15. Verfahren zur Herstellung einer Tablette auf Basis eines Progestins, vorzugsweise von Progesteron, **dadurch gekennzeichnet, dass** man
- ein erstes Gemisch aus Progestin und Tensid bereitet,
- dieses erste Gemisch mikronisiert, um ein mit dem Tensid co-mikronisiertes Progestin zu erhalten,
- eine Anfeuchtlösung bereitet,
- das mit dem Tensid co-mikronisierte Progestin mit der Anfeuchtlöstung befeuchtet,
- das erhaltene Gemisch zu Granulaten granuliert,
- die Granulate trocknet und danach kalibriert,
- den getrockneten und kalibrierten Granulaten Sprengmittel, Verdünnungsmittel, Färbemittel, Gleitmittel zusetzt,
- dieses Gemisch zu Tabletten presst.

16. Verfahren nach Anspruch 15, **dadurch gekennzeichnet, dass** das Tensid Natriumlaurylsulfat ist.

17. Verwendung eines mit einem Tensid co-mikronisierten Progestins nach einem der Ansprüche 1 bis 4 oder von mit einem Tensid co-mikronisiertem Progesteron nach Anspruch 5 zur Herstellung eines Medikaments zur Behandlung eines mit einer ungenügenden Progesteronsekretion verbundenen physiologischen Zustandes, wie der Menopause.

## Claims

1. A progestin co-micronized with a surfactant.

2. The co-micronized progestin as claimed in claim 1,
**characterized in that** the progestin is progesterone.

3. The co-micronized progestin as claimed in either of claims 1 and 2, **characterized in that** the surfactant is sodium lauryl sulfate.

4. The co-micronized progestin as claimed in any one of claims 1 to 3, **characterized in that** it exhibits a progestin content of between 80.0% and 99.9%, preferably between 90.0% and 99.5%, and even more preferentially between 95.0% and 99.0%, and a surfactant content of between 0.1% and 20.0%, preferably between 0.5% and 10.0%, and even more preferentially between 1.0% and 5.0%, the percentages being expressed by weight of solids.

5. A progesterone co-micronized with a surfactant, preferably sodium sodium lauryl sulfate, **characterized in that** the surfactant/progesterone ratio is between 1/200 and 1/20, preferably between 1/150 and 1/40.

6. A pharmaceutical composition comprising the progestin co-micronized with a surfactant as claimed in any one of claims 1 to 4.

7. A pharmaceutical composition comprising the progesterone co-micronized with a surfactant as claimed in claim 5.

8. The pharmaceutical composition as claimed in either of claims 6 and 7, **characterized in that** it exhibits a surfactant content of between 0.001% and 5%, preferably between 0.002% and 3%, and even more preferentially between 0.005% and 2%, relative to the total solids of the composition.

9. The pharmaceutical composition as claimed in claim 7, **characterized in that** it exhibits a surfactant content of between 0.1% and 5%, preferably between 0.2% and 3%, even more preferentially between 0.2% and 2%, by weight relative to the total solids of the composition.

10. The pharmaceutical composition as claimed in any one of claims 6 to 9, **characterized in that** it is in the form of a soft capsule, of a gelatine capsule, of a tablet, or of a lyophilisate, or of a powder or of a granule, including microgranules.

11. The pharmaceutical composition as claimed in any one of claims 6 to 10, **characterized in that** it contains, in addition to the co-micronized progestin, an estrogen or one of its derivatives.

12. The pharmaceutical composition as claimed in claim 11, **characterized in that** the estrogen is selected from the group consisting of 17β-estradiol, estrone, 17α-ethinylestradiol and estradiol valerate, preferably 17β-estradiol.

13. The pharmaceutical composition as claimed in either of claims 11 and 12, **characterized in that** it exhibits an estrogen content of between 0.05% and 5%, preferably between 0.1% and 3%.

14. A method for co-micronization of a progestin with a surfactant, **characterized in that** a mixture of a progestin with a surfactant is prepared, and then grinding of this mixture is subsequently carried out using an airjet mill, a colloidal mill or a ball mill.

15. A method for preparing a tablet based on a progestin, preferably progesterone, **characterized in that** :
- a first mixture of progestin and surfactant is prepared,
- this first mixture is micronized in order to obtain a progestin co-micronized with the surfactant,
- a wetting solution is prepared,
- the progestin co-micronized with the surfactant is wetted with the wetting solution,
- the mixture obtained is granulated in order to obtain granules,
- the granules are dried and are then calibrated,
- disintegrating agents, diluents, dyes, lubricants are added to the dried and calibrated granules,
- this mixture is compressed in order to obtain tablets.

16. The method of preparation as claimed in claim 15,
**characterized in that** the surfactant is sodium lauryl sulfate.

17. The use of a progestin co-micronized with a surfactant as claimed in any one of claims 1 to 4, or of the progesterone co-micronized with a surfactant as claimed in claim 5, for preparing a medicinal product intended for the treatment of a physiological condition related to insufficiency of progesterone secretion, such as menopause.
